# EUROPEAN PATENT APPLICATION

(11) **EP 4 670 664 A1**
(43) Date of publication of application: **31.12.2025**
(21) Application number: 25180921.6
(22) Date of filing: 05.06.2025
(51) Int. Cl.: A61B 34/20, A61B 34/30

(54) **SURGICAL NAVIGATION SYSTEMS AND METHODS FOR DETECTING LOCALIZER MOTION**

(30) Priority: 28.06.2024 US 202463665805 P
(71) Applicant: MAKO Surgical Corp., Weston, FL 33331 (US)
(72) Inventor: Talasaz, Ali, Plantation, 33325-2514 (US)
(74) Representative: Schott, Jakob Valentin

(57) **Abstract**

Systems and methods for detecting localizer motion are provided. One such system is a surgical navigation system which includes a first tracker, a second tracker, a localizer, and one or more controllers. The localizer is configured to track the first tracker to generate first tracking data, and to track the second tracker to generate second tracking data. The one or more controllers are configured to receive the first tracking data and the second tracking data, calculate a relative motion of the first tracker based on the first tracking data, calculate a relative motion of the second tracker based on the second tracking data, compare the relative motions of the first and second trackers to one another, and detect a movement of the localizer based on the comparison.

## Description

### CROSS-REFERENCE TO RELATED APPLICATIONS

The subject application claims priority to and all the benefits of United States Provisional Patent App. No. 63/665,805, filed June 28, 2024, the entire contents of which are hereby incorporated by reference.

### BACKGROUND

Surgical navigation systems assist users in tracking/locating objects in an operating room. For instance, navigation systems assist surgeons in placing surgical instruments relative to a patient's anatomy. Typically, the tool and the anatomy are tracked together with their relative movement shown on a display. Often the navigation system includes tracking devices attached to the object being tracked, and a localizer determines positions of the tracking devices to determine a position and/or orientation of the object. The navigation system then monitors movement of the objects via the tracking devices, such as by determining the location of these tracking devices relative to a coordinate system associated with the localizer. As such, issues may arise if the localizer moves while keeping track of the tracking devices, especially if this movement is undetectable by the system.

### SUMMARY

According to a first aspect, a surgical navigation system is provided. The surgical navigation system includes a first tracker, a second tracker, a localizer, and one or more controllers. The localizer is configured to track the first tracker to generate first tracking data and to track the second tracker to generate second tracking data. The one or more controllers are configured to receive the first tracking data and the second tracking data, calculate a relative motion of the first tracker based on the first tracking data, calculate a relative motion of the second tracker based on the second tracking data, compare the relative motions of the first and second trackers to one another, and detect a movement of the localizer based on the comparison.

According to a second aspect, a method of operating a surgical navigation system which includes a first tracker, a second tracker, and a localizer configured to track the first tracker and the second tracker is provided. The method includes receiving a first tracking data associated with the first tracker from the localizer, receiving a second tracking data associated with the second tracker from the localizer, calculating a relative motion of the first tracker based on the first tracking data, calculating a relative motion of the second tracker based on the second tracking data, comparing the relative motions of the first and second trackers to one another, and detecting a movement of the localizer based on the comparison.

According to a third aspect, a surgical navigation system is provided. The surgical navigation system includes a first tracker, a second tracker, a localizer, and one or more controllers. The localizer is configured to track the first tracker to generate first tracking data, and to track the second tracker to generate second tracking data. The one or more controllers are configured to receive the first tracking data and the second tracking data, and to determine a movement of the localizer based on the first tracking data and the second tracking data.

Any of the above aspects can be combined in part or in whole with any other aspect. Any of the above aspects, whether combined in part or in whole, can be further combined with any of the following implementations, in full or in part.

The relative motions of the trackers and the localizer may be more specifically defined and calculated. In some implementations, the relative motion of the first tracker, the relative motion of the second tracker, and the movement of the localizer may each include at least one of a speed, a velocity, and an acceleration. In some implementations, the relative motion of the first tracker may include a first tracker velocity pattern, the relative motion of the second tracker may include a second tracker velocity pattern, and the one or more controllers are configured to compare the first tracker velocity pattern to the second tracker velocity pattern to determine the movement of the localizer. In some implementations, the one or more controllers are configured to calculate a first tracker velocity based on the first tracking data, calculate a second tracker velocity based on the second tracking data, compare the first tracker velocity to the second tracker velocity, and determine the movement of the localizer based on the comparison.

The relative motion of the trackers and the movement of the localizer may be more specifically calculated/determined. In some implementations, the relative motion of the first tracker may realized as a first tracker motion parameter, the relative motion of the second tracker may be realized as a second tracker motion parameter, the movement of the localizer may be realized as a localizer motion parameter. In such implementations, the one or more controllers may be configured to compare the first tracker motion parameter to the second tracker motion parameter to calculate a similarity metric, and to determine the localizer motion parameter based on the first tracker motion parameter, the second tracker motion parameter, and the similarity metric. Further, the similarity metric may be realized as a first similarity metric, the first tracker motion parameter may include a first tracker velocity at a first time and a first tracker velocity at a second time, the second tracker motion parameter may include a second tracker velocity at the first time and a second tracker velocity at the second time. Even further, the one or more controllers may be configured to compare the first tracker velocity at the first time to the second tracker velocity at the first time to calculate the first similarity metric, compare the first tracker velocity at the second time to the second tracker velocity at the second time to calculate a second similarity metric, and determine the localizer motion parameter based on the first tracker motion parameter, the second tracker motion parameter, the first similarity metric, and the second similarity metric. In some implementations, the one or more controllers may be configured to calculate a similarity metric by subtracting the second tracker velocity from the first tracker velocity, and determine the movement of the localizer based on the first tracker velocity, the second tracker velocity, and the similarity metric.

In addition to tracking data generated by the localizer, kinematic data generated by a robotic manipulator may also be used to determine movement of the localizer. In some implementations, the movement of the localizer may be realized as a localizer motion parameter, the first tracker may be coupled to a robotic manipulator, the relative motion of the first tracker may be realized as a manipulator motion parameter, and the one or more controllers are configured to receive kinematic data from the robotic manipulator, and calculate a kinematic motion parameter based on the kinematic data. In some implementations, the one or more controllers are configured to receive kinematic data from the robotic manipulator, calculate a kinematic motion parameter based on the kinematic data, determine at least one localizer parameter corresponding to the localizer, and calculate the relative motion of the first tracker based on the first tracking data and the at least one localizer parameter. In some implementations, the at least one localizer parameter may be a latency period. In such implementations, the one or more controllers may be configured to calculate the kinematic motion parameter based on kinematic data stored in memory at a first time, and calculate the relative motion of the first tracker based on the first tracking data stored in memory at a second time. The second time may be after the first time by a time period equal to the latency period.

The tracking data and/or the kinematic data may be filtered. In some implementations, the one or more controllers may be configured to apply at least one filtering algorithm to the first tracking data to determine the relative motion of the first tracker, and/or apply the at least one filtering algorithm to the second tracking data to determine the relative motion of the first tracker. In some implementations, the one or more controllers may be configured to receive kinematic data from the robotic manipulator, calculate a kinematic motion parameter based on the kinematic data, apply at least one filtering algorithm to the first tracking data to determine the relative motion of the first tracker, and apply the at least one filtering algorithm to the kinematic data to determine a kinematic motion parameter. In such implementations, the at least one filtering algorithm may be applied to the first tracking data and the kinematic data using identical filter settings to ensure that the tracking data is filtered in the same way as the kinematic data. The at least one filtering algorithm may include at least one of a derivative filtering algorithm, and a moving average filtering algorithm.

The localizer motion parameter may be scaled and/or normalized in order to increase the range of tracker motion that may be used to determine movement of the localizer. In some implementations, the one or more controllers are configured to scale the movement of the localizer based on the relative motion of the second tracker. In some implementations, the localizer motion parameter may be scaled based on the anatomy motion parameter, such as in response to a determination that the anatomy tracker is within the field of view of the localizer. In some implementations, the one or more controllers are configured to determine an anatomy motion threshold, calculate an anatomy motion ratio equal to the anatomy motion parameter divided by the anatomy motion threshold, and scale the localizer motion parameter by the anatomy motion ratio. In some implementations, the localizer motion parameter may be normalized based on at least one of the kinematic motion parameter and the manipulator motion parameter. In some implementations, the one or more controllers may normalize the localizer motion parameter by being configured to divide the localizer motion parameter by one of: the kinematic motion parameter, the manipulator motion parameter, or an average of the kinematic motion parameter and the manipulator motion parameter.

Various warning may be triggered during operation of the systems or while carrying out of the methods. In some implementations, the one or more controllers may be configured to trigger a warning if the movement of the localizer is above a threshold localizer motion parameter. In some implementations, the relative motion of the second tracker may include a second tracker velocity, and the one or more controllers are configured to trigger a warning if the second tracker velocity is above a threshold velocity. In some implementations, the localizer may include a field of view, the second tracker may be realized as an anatomy tracker and may be coupled to a bone of a patient, the relative motion of the second tracker may be realized as an anatomy motion parameter, and the one or more controllers are configured to determine whether the anatomy tracker is within the field of view of the localizer based on the second tracking data, and trigger a warning in response to a determination that the anatomy tracker is outside of the field of view of the localizer. The warning(s) may include at least one of a visual on a display, an audible alert, and a command sent to a robotic manipulator or surgical instrument.

### BRIEF DESCRIPTION OF THE DRAWINGS

Advantages of the present invention will be readily appreciated as the same becomes better understood by reference to the following detailed description when considered in connection with the accompanying drawings.
Figure 1 is a perspective view of one implementation of a surgical system comprising a robotic manipulator and a navigation system including a localizer.
Figure 2 is a perspective view illustrating transforms between a manipulator, a patient, and a navigation system, according to one embodiment of the invention.
Figure 3 shows a flowchart describing a method of detecting movement of a localizer, according to one implementation.
Figure 4 shows a flowchart describing a method of detecting movement of a localizer based on tracking data, according to one implementation.
Figure 5 shows a flowchart describing a method of detecting movement of a localizer based on tracking data and/or kinematic data, according to one implementation.

### DETAILED DESCRIPTION

### I. Example System Overview

Referring to the Figures, wherein like numerals indicate like or corresponding parts throughout the several views, a surgical navigation system (hereinafter "system") and method for operating the same are shown throughout.

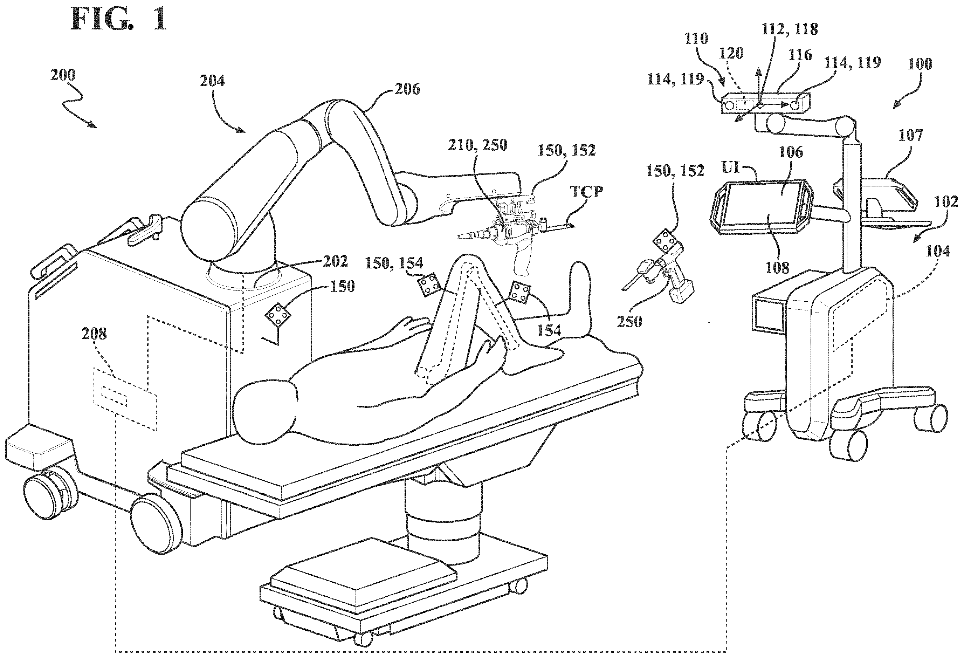

Referring to Figure 1, an example configuration of an operation room or surgical suite for performing a medical procedure on a patient is shown. The illustrated configuration includes a surgical navigation system 100, a surgical robot 200, a surgical instrument 250, and an implant IM to be implanted into the patient. The surgical navigation system 100 is set up to track movement of various objects in the operating room. Such objects include, for example, the patient, the surgical robot 200, and/or the surgical instrument 250, among other objects. The surgical navigation system 100 tracks these objects for purposes of displaying their relative positions and orientations to the surgeon and, in some cases, for purposes of controlling or constraining movement of the surgical instrument 250 relative to virtual cutting boundaries associated with the patient.

The surgical navigation system 100 may include a computer cart assembly 102 that houses a navigation computer 104. A navigation interface is in operative communication with the navigation computer 104. The navigation interface includes a first display 106 adapted to be situated outside of the sterile field and a second display 107 adapted to be situated inside the sterile field. The displays 106, 107 are adjustably mounted to the computer cart assembly 102. First and second input devices (not shown) such as a keyboard and mouse can be used to input information into the navigation computer 104 or otherwise select/control certain aspects of the navigation computer 104. Other input devices are contemplated including a touch screen 108, gesture control, or voice-activation. The displays can be implemented as head-mounted displays configured for extended reality or augmented reality and adapted to display any of the graphics or imagery described herein in manner that is overlaid or superimposed over real-world views or video.

Further, the navigation system 100 includes a localizer 110 in communication with the navigation computer 104. In the illustrated implementation, the localizer 110 is a multi-modality localizer and includes an optical (video) camera unit 112 and an infrared and/or near-infrared (NIR) sensor unit 114. The optical camera unit 112 includes one or more sensors 118 that are adapted to sense light in the visible spectrum and may be configured as a video camera or machine vision or computer vision system. The visible light sensors 118 are configured to detect color and or produce data that can be used to create depth maps. The infrared sensor unit 114 may include one or more sensors 119 that are adapted to sense light in the infrared or near-infrared spectrum. The localizer 110 may include illuminators that are configured to radiate infrared light into the surgical field to enable the infrared sensors 119 to detect reflected or backscatter radiation. An outer casing 116 that houses the optical camera unit 112 and the infrared sensor unit 114.

The localizer 110 is in communication with the navigation computer 104. In some implementations, a camera controller 120 facilitates communication between the sensors 118, 119 and the navigation computer 104 through either a wired or a wireless connection (not shown). In other implementations, the sensors 118, 119 may communicate directly with the navigation computer 104. Processing of the signals from the visible light sensor(s) 118 and the IR sensor(s) 119 may occur at the navigation computer 104 for processing both navigation and machine vision information. One example of the navigation system 100 is described in U.S. Patent No. 9,008,757, entitled, "Navigation System Including Optical and Non-Optical Sensors," hereby incorporated by reference.

The navigation computer 104 can be a personal computer or laptop computer. The navigation computer 104 may have the display 106, central processing unit (CPU) and/or other processors, memory (not shown), and storage (not shown). The navigation computer 104 may be loaded with software which converts the signals received from the camera unit 112 and the infrared sensor unit 114 into data representative of the position and orientation of the objects being tracked. Additionally, the software converts the signals received from the camera unit 112 into data that can identify the objects, such as through object recognition from the optical camera unit 112. Position and orientation signals and/or data are transmitted to the navigation computer 104 for purposes of tracking objects. In an alternative, all of the computer processing components and functionality may be integrated into a single processing units or may be distributed between or among multiple processing units. Moreover, although described as taking place at a particular computer or controller in the present disclosure, it will be appreciated by one of skill in the art that any processing tasks may take place or be performed by other computers or controllers. The computer cart assembly 102, display 106, and camera unit 112 may be like those described in U.S. Pat. No. 7,725,162 to Malackowski, et al. issued on May 25, 2010, entitled "Surgery System," hereby incorporated by reference.

The surgical navigation system 100 may be used to track the poses of a plurality of tracking devices, herein referred to as trackers 150. In the illustrated implementation, the trackers 150 include a manipulator tracker 152 coupled to the surgical robot 200 (or the instrument 250 coupled thereto), an anatomy tracker 154 coupled to a first anatomical position of the patient (e.g., a first bone), and another anatomy tracker 154 coupled to a second anatomical position of the patient (e.g., the first bone, a second bone, etc.). Other trackers 150 are contemplated.

The trackers 150 may be active trackers or passive trackers. Active trackers require a power source and have an array of fiducials (also referred to as tracking elements or markers) that actively generate and emit radiation in a wavelength detectable by the visible light sensor 118. The fiducials of an active tracker may be a light emitting diode (LED), including, for example, an infrared LED. The array of active fiducials may be "always on" or may be operative to selectively fire, that is emit radiation, according to and in response to commands from the surgical navigation system 100. In such selective-fire active trackers, the tracker may communicate by way of a wired or a wireless connection with the navigation computer 104 of surgical navigation system 100. In other examples, the active trackers can include active electromagnetic elements, active radio-frequency elements, and the like.

In alternative implementations, the trackers 150 may include passive trackers. The active tracker may be battery powered with an internal battery or may have leads to receive power through the navigation computer 104, which may receive external power. The passive tracker array typically does not require a power source. Passive trackers can include barcodes, QR codes, or any computer-detectable pattern. Passive trackers can include passive reflective markers, radio-opaque markers, passive electromagnetic elements, passive radio-frequency elements, and the like.

In some examples, the navigation system 100 and/or the localizer 110 are radio frequency (RF) based. For example, the navigation system 100 may comprise an RF transceiver coupled to the navigation computer 104. Here, the trackers 150 may comprise RF emitters or transponders, which may be passive or may be actively energized. The RF transceiver transmits an RF tracking signal, and the RF emitters respond with RF signals such that tracked states are communicated to (or interpreted by) the navigation computer 104. The RF signals may be of any suitable frequency. The RF transceiver may be positioned at any suitable location to track the objects using RF signals effectively. Furthermore, examples of RF-based navigation systems may have structural configurations that are different than the navigation system 100 illustrated throughout the drawings.

In some examples, the navigation system 100 and/or localizer 110 are electromagnetic (EM) based. For example, the navigation system 100 may comprise an EM transceiver coupled to the navigation computer 104. Here, the trackers 150 may comprise EM components attached thereto (e.g., various types of magnetic trackers, electromagnetic trackers, inductive trackers, and the like), which may be passive or may be actively energized. The EM transceiver generates an EM field, and the EM components respond with EM signals such that tracked states are communicated to (or interpreted by) the navigation computer 104. The navigation computer 104 may analyze the received EM signals to associate relative states thereto. Here too, examples of EM-based navigation systems may have structural configurations that are different than the navigation system 100 illustrated throughout the drawings.

In some examples, the navigation system 100 and/or the localizer 110 could be based on one or more other types of tracking systems. For example, an ultrasound-based tracking system coupled to the navigation computer 104 could be provided to facilitate acquisition of ultrasound images of markers that define trackable features such that tracked states are communicated to (or interpreted by) the navigation computer 104 based on the ultrasound images. By way of further example, a fluoroscopy-based imaging system (e.g., a C-arm) coupled to the navigation computer 104 could be provided to facilitate acquisition of X-ray images of radio-opaque markers that define trackable features such that tracked states are communicated to (or interpreted by) the navigation computer 104 based on the X-ray images.

Furthermore, in some examples, a machine-vision tracking system (e.g., one or more charge-coupled devices) coupled to the navigation computer 104 could be provided to facilitate acquiring 2D and/or 3D machine-vision images of structural features that define trackable features such that tracked states are communicated to (or interpreted by) the navigation computer 104 based on the machine-vision images. The ultrasound, X-ray, and/or machine-vision images may be 2D, 3D, or a combination thereof, and may be processed by the navigation computer 104 in near real-time to determine the tracked states of the trackers 150.

Various types of tracking and/or imaging systems could define the localizer 110 and/or form a part of the navigation system 100 without departing from the scope of the present disclosure. Furthermore, the navigation system 100 and/or localizer 110 may have other suitable components or structure not specifically recited herein, and the various techniques, methods, and/or components described herein with respect to the optically based navigation system 100 shown throughout the drawings may be implemented or provided for any of the other examples of the navigation system 100 described herein. For example, the navigation system 100 may utilize solely inertial tracking and/or combinations of different tracking techniques, sensors, and the like. Other configurations are contemplated.

The navigation system 100 may be used to track the surgical robot 200 as noted above. In some implementations, the surgical robot 200 includes a base 202 and a manipulator 204 including a plurality of links and joints. The base 202 may be fixed to a point in the operating room, such as the operating table. Alternatively, the base 202 may be readily movable such that the surgical robot can be repositioned in the operating room. In one example, the surgical robot 200 can have a configuration such as the robotic manipulator described in US Patent No. 10,327,849, entitled "Robotic System and Method for Backdriving the Same", the contents of which are hereby incorporated by reference in its entirety.

The surgical robot 200 may house a manipulator controller 208, or other type of control unit. The manipulator controller 208 may comprise one or more computers, or any other suitable form of controller that directs the motion of the manipulator 204 and/or the base 202. The manipulator controller 208 may have a central processing unit (CPU) and/or other processors, memory, and storage. The processors could include one or more processors to control operation of the manipulator 204. The processors can be any type of microprocessor, multi-processor, and/or multi-core processing system. The manipulator controller 208 may additionally, or alternatively, comprise one or more microcontrollers, field programmable gate arrays, systems on a chip, discrete circuitry, and/or other suitable hardware, software, or firmware that is capable of carrying out the functions described herein. The term processor is not intended to limit any implementation to a single processor. The surgical robot 200 may also comprise a user interface UI with one or more displays and/or input devices (e.g., push buttons, keyboard, mouse, microphone (voice-activation), gesture control devices, touchscreens, etc.).

The surgical robot 200 may be used to control the surgical instrument 250. To that end, the surgical robot 200 may include an end effector 210 configured to couple the surgical instrument 250 to the manipulator 204. In some implementations, the manipulator 204 and the instrument 250 may be arranged like that shown in U.S. Patent No. 9,566,121, filed on March 15, 2014, entitled, "End Effector of a Surgical Robotic Manipulator," hereby incorporated by reference. In other implementations, the surgical instrument 250 is attached to the manipulator 204 as shown in U.S. Pat. No. 9,119,655, issued Sep. 1, 2015, entitled, "Surgical Manipulator Capable of Controlling a Surgical Instrument in Multiple Modes", the disclosure of which is hereby incorporated by reference.

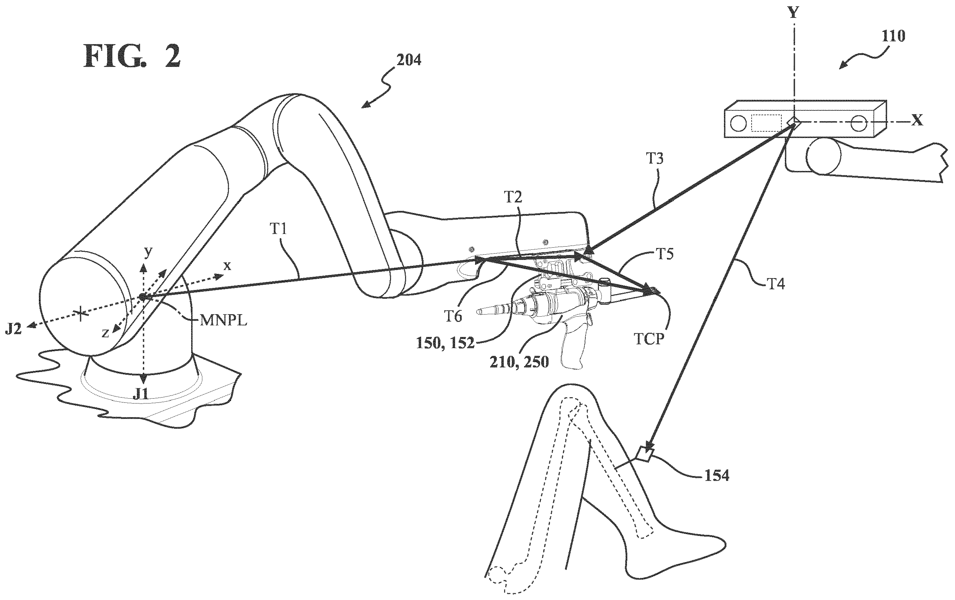

Referring to Figure 2, the state of one component of the system 10 relative to the state of another component is represented as a transform (shown in the figures using arrows). In one embodiment, each transform is specified as a transformation matrix, such as a 4×4 homogenous transformation matrix. The transformation matrix, for example, includes three unit vectors representing orientation, specifying the axes (X, Y, Z) from the first coordinate system expressed in coordinates of the second coordinate system (forming a rotation matrix), and one vector (position vector) representing position using the origin from the first coordinate system expressed in coordinates of the second coordinate system. The transform, when calculated, gives the state (position and/or orientation) of the component from the first coordinate system given with respect to a second coordinate system. At least one of the controllers 104, 208 calculates/obtains and combines a plurality of transforms e.g., from the various components of the system 10 to control the manipulator 204, as described below. For brevity, although either of (or both of) the controllers 104, 208 may be utilized, the navigation computer 104 is herein described as determining the pose of the manipulator 204 and/or the TCP of the end effector 210 as well as merging data from the navigation computer 104 and the manipulator controller 208 as further described below.

As shown in Figure 2, the transforms include the following: a first transform T1 between the base 202 and the end effector 210, a second transform T2 between the end effector 210 and the manipulator tracker 152 , a third transform T3 between the localizer 110 and the manipulator tracker 152, a fourth transform T4 between the localizer 110 and one or more of the patient trackers 154, a fifth transform T5 between the manipulator tracker 152 and the TCP, and a sixth transform T6 between the end effector 210 and the TCP.

The transforms are now described in detail. The computer 104 acquires raw kinematic measurement data relating to a state of the end effector 210. The state of the end effector 210 may be determined relative to the manipulator coordinate system MNPL. In some instances, the raw kinematic measurement data may relate to the state of the end effector 210 relative to the base 202. The raw kinematic measurement data may be obtained from the manipulator controller 208. Specifically, as shown in Figure 2, the computer 104 is configured to acquire the raw kinematic measurement data by acquiring one or more values of the first transform T1 between a state of the base 202 and the state of the end effector 210. Here, the raw kinematic measurement data may be obtained from kinematic data of the manipulator 204. In particular, the computer 104 may acquire one or more values of the first transform T1 by applying a forward kinematic calculation to values acquired from the joint encoders 19. Thus, the state of the end effector 210 can be determined relative to the manipulator coordinate system MNPL without intervention from the navigation system 32. In other words, the first transform T1 may be obtained irrespective of any measurements from the navigation system 32.

In Figure 2, the first transform T1 is indicated by an arrow having an origin at the base 202 and extending to and having an arrowhead pointing to the end effector 210. In one exemplary convention used throughout Figure 2, the arrowhead points to the component having its state derived or specified relative to the component at the origin. The first transform T1 may be determined using any suitable reference frames (coordinate systems) on the base 202 and the end effector 210.

The computer 104 may further acquire known relationship data relating to the state of the manipulator tracker 152 relative to the end effector 210. In general, the known relationship data may be derived from any known relationship between the manipulator tracker 152 and the end effector 210. In other words, the manipulator tracker 152 and the end effector 210 have a relationship that is known or calculatable using any suitable method. The manipulator tracker 152 and the end effector 210 may be fixed or moving relative to each other. For example, the manipulator tracker 152 may be attached directly to the end effector 210, as shown in Figure 2. Alternatively, the manipulator tracker 152 may be attached to one of the links 18, which move relative to the end effector 210. In general, the manipulator tracker 152 and the end effector 210 are tracked by different techniques, i.e., by navigation data and kinematic measurement data, respectively. The known relationship data assists to bridge the kinematic measurement data and the navigation data by aligning the manipulator tracker 152 and the end effector 210 to a common coordinate system.

The known relationship data may be fixed (constant or static) or variable. In embodiments where the known relationship data is fixed, the known relationship data may be derived from calibration information relating to the manipulator tracker 152 and/or the end effector 210. For example, the calibration information may be obtained at a manufacturing/assembly stage, e.g., using coordinate measuring machine (CMM) measurements, etc. The known relationship data may be obtained using any suitable method, such as reading the known relationship data from a computer-readable medium, an RFID tag, a barcode scanner, or the like. The known relationship data may be imported into system 10 at any suitable moment such that the known relationship data is readily accessible by the computer 104. In embodiments where the known relationship data is variable, the known relationship data may be measured or computed using any ancillary measurement system or components, such as additional sensors, trackers, encoders, or the like. The known relationship data may also be acquired after mounting the manipulator tracker 152 to the end effector 210 in preparation for a procedure by using any suitable technique or calibration method.

Whether static or variable, the known relationship data may or may not be regarded as raw data, as described herein, depending on the desired technique for obtaining the same. In one embodiment, the computer 104 may acquire the known relationship data by acquiring one or more values of the second transform T2 between the state of the manipulator tracker 152 and the state of the end effector 210. The second transform T2 may be determined with respect to any suitable coordinate system or frame on the manipulator tracker 152 and the end effector 210.

In other embodiments, the computer 104 may determine the second transform T2 using any one or more of kinematic measurement data from the manipulator 204 and navigation data from the navigation system 32 such that known relationship data is not utilized. For example, the second transform T2 may be calculated using one or more of raw kinematic measurement data relating to the state of the end effector 210 relative to the manipulator coordinate system MNPL from the manipulator 204 and raw navigation data relating to the state of the tracker 152 relative to the localizer 110 from the navigation system 32. For example, the end effector 210 may be rotated about its wrist to create a circular or spherical fit of the end effector 210 relative to the manipulator tracker 152.

In some embodiments, it may be desirable to determine the state of the TCP relative to the manipulator coordinate system MNPL and/or the localizer coordinate system LCLZ. For example, the computer 104 may further acquire known relationship data relating to the state of the end effector 210 relative to the TCP by acquiring one or more values of the sixth transform T6, as shown in Figure 2. Additionally, or alternatively, the computer 104 may acquire known relationship data relating to the state of the manipulator tracker 152 relative to the TCP by acquiring one or more values of the fifth transform T5, as shown in Figure 2. The fifth and sixth transforms T5, T6 may be acquired using any of the aforementioned techniques described with respect to transform T2. The fifth and sixth transforms T5, T6 may be utilized to determine commanded states of the TCP relative to the surgical site, as well as actual states of the same, as described in the subsequent section.

The fourth transform T4 between the localizer 110 and one or more of the patient trackers 154 may be determined by the computer 104 by similar techniques and assumptions as described above with respect to the other transforms T1-T6. Specifically, the localizer 110 is configured to monitor the state of one or more of the patient trackers 154 and the computer 104 is configured to acquire, from the navigation system 32, raw navigation data relating to the state of the one or more of the patient trackers 154 relative to the localizer 110.

The first and sixth transforms T1, T6 may be utilized by the computer 104 to determine the location of the TCP relative to the manipulator coordinate system MNLP as described above. As the first transform T1 is based on kinematic data, location of the TCP relative to the manipulator coordinate system MNPL is also based on the kinematic data. Additionally, the third and fifth transforms T3, T5 may be utilized by the computer 104 to determine the location of the TCP relative to the localizer coordinate system LCLZ. As the third and fifth transforms T3, T5 are based on manipulator tracking data, the location of the TCP relative to the localizer coordinate system LCLZ is also based on the manipulator tracking data. The computer 104 may utilize one or both of the kinematic data and tracking data to ensure accuracy of at least one of the tracking methods (i.e. optical tracking via the localizer and kinematic tracking via the joint encoders).
II. Example Methods for Detection of Localizer Motion

During surgery, a loss of accuracy of the surgical navigation system 100 can lead to imprecise instrument positioning and potentially cause complications for the surgeon and/or the patient. One vector of accuracy loss is movement of the localizer 110, which causes the localizer coordinate system LCLZ to move relative to the trackers 150 and the manipulator tracking system MNPL. For example, in many modern surgical navigation systems, at least one of the displays 106, 107 includes a touch-sensitive input arrangement, such as the touch screen 108. And as will be appreciated from Figure 1, inputs to the touch screen 108 can cause the entire computer cart assembly 102 to move slightly. Since the localizer 110 of the illustrated implementation is also attached to the cart assembly 102, the localizer 110 may also move slightly in response to the user touching the touch screen 108. Unintentional movement of the localizer 110 (or other tracking device) may occur in other ways as well, such as the user bumping into the localizer 110 or cart assembly 102. Regardless of the cause, the methods described below may be carried out by the surgical navigation system 100 in order to detect the unintentional movement of the localizer 110, and/or to alert the user of said movement.

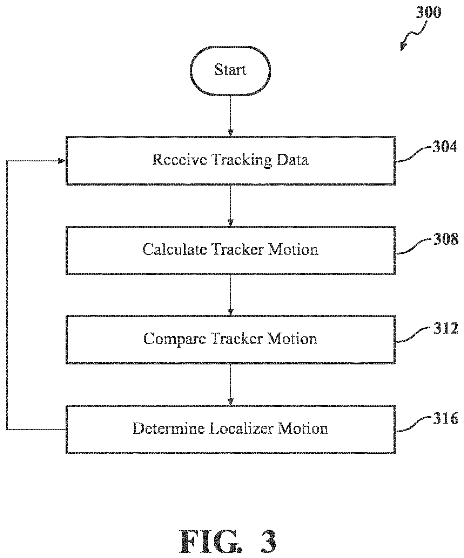

Referring to Figure 3, a flowchart describing a method 300 for detecting motion of the localizer 110 according to one implementation is shown. The method 300 may be carried out by one or more controllers and has been described below as being carried out by the navigation computer 104 for brevity.

At 304, tracking data is received. In some implementations, the localizer 110 may generate the tracking data by tracking at least two of the trackers 150, and the tracking data may be received by the navigation computer 104. For example, the localizer 110 may generate a first tracking data by tracking the manipulator tracker 152 and generate a second tracking data by tracking the anatomy tracker 154. After which, the localizer 110 may transmit the first and second tracking data to the navigation computer 104.

At 308, a relative motion of each tracker 150 is calculated based on the tracking data received at step 304. For example, the relative motion of the trackers 150 may include the motion of the trackers 150 relative to the localizer 110 and/or relative to a specified tracker 150. In some implementations, the relative motion of each tracker 150 is calculated as a tracker motion parameter, which is a motion parameter associated with the corresponding tracker 150. The tracker motion parameters may include at least one of a speed, a velocity, and an acceleration of the trackers associated with the tracking data received at 304. Continuing with the above example, the navigation computer 104 may calculate a first tracker motion parameter and a second tracker motion parameter based on the first tracking data and the second tracking data, respectively. More specifically, the navigation computer 104 may calculate a first tracker velocity associated with the manipulator tracker 152 and a second tracker velocity associated with the anatomy tracker 154. In some implementations, the localizer 110 may perform these calculations and send the tracker motion parameters to the navigation computer 104.

At 312, the relative motions of each tracker 150 motion parameters are compared to one another. For example, the first tracker motion parameter may be compared to the second tracker motion parameter. At 316, a movement of the localizer 110 is determined based on the comparison performed at 312. For example, a localizer motion parameter may be determined based on the comparison performed at 312. Again, continuing with the previous example, the first tracker motion parameter/manipulator tracker velocity may be compared to the second tracker motion parameter/anatomy tracker velocity. Depending on the similarity between the velocities of the manipulator tracker 152 and the anatomy tracker 154, the navigation computer 104 may determine that the localizer 110 has moved. Expanding on the example, if the manipulator tracker velocity was 5 mm/s in a first direction and the anatomy tracker velocity was also 5 mm/s in the first direction, this may be indicative of the localizer 110 having moved at 5 mm/s in a second direction. Thus, at 316, the navigation computer 104 may determine that the localizer motion parameter was 5 mm/s in the second direction. Alternatively, if the anatomy tracker velocity was stationary, and the manipulator tracker velocity was 5mm/s is the second direction, this may be indicative of the manipulator 204 having moved rather than the localizer 110 having moved. In general, the higher the similarity between the relative motions of the trackers 152, 154 (e.g., tracker velocities), the higher the likelihood that the localizer 110 moved.

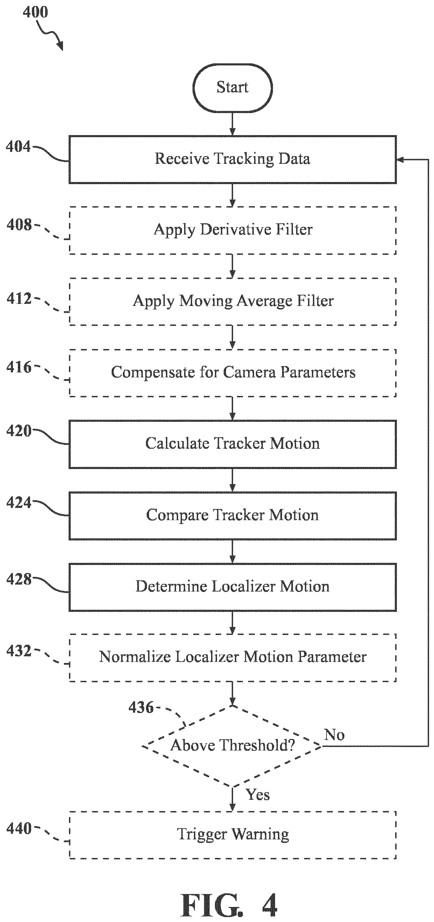

Referring to Figure 4, a flowchart describing a method 400 for detecting movement of the localizer 110 according to another implementation is shown. The method 400 is similar to the previously described method 300, but a number of optional steps are included in addition to the other steps 304-316. At 404, like at 304 from the previous method 300, tracking data is received. Again, the localizer 110 may generate the tracking data by tracking at least two of the trackers 150, and the tracking data may be received by the navigation computer 104. After 404, however, a number of new (optional) steps are included prior to the relative motion of each tracker 150 (e.g., the tracker motion parameters) being calculated.

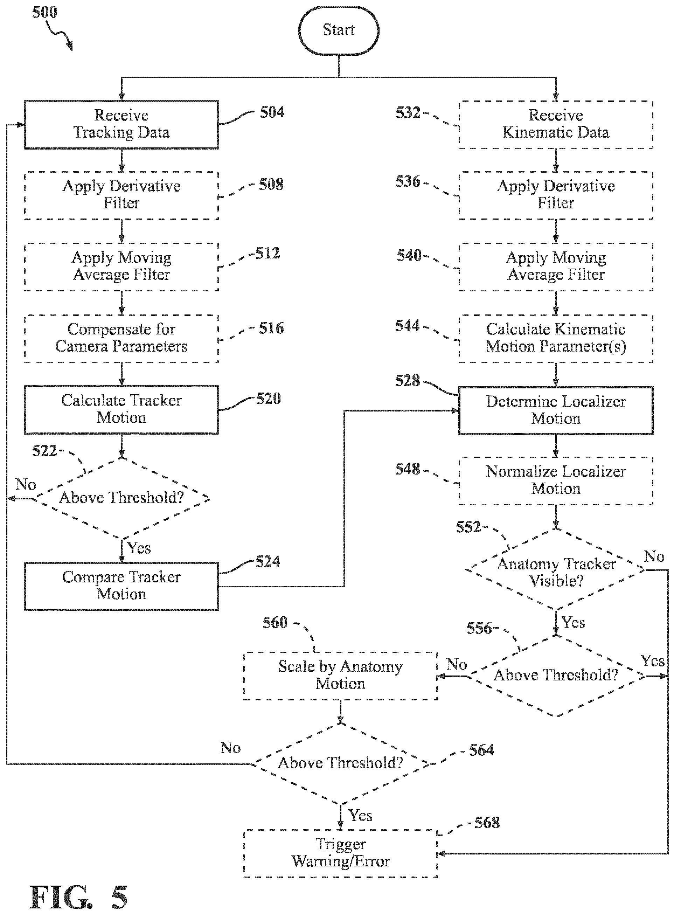

A series of operations may be performed on the tracking data to aid in calculating the relative motion of each tracker 150. For example, at least one filtering algorithm may be applied to the tracking data. At 408, a derivative filter may be applied to the tracking data. In some implementations, the tracking data corresponds to a series of positions of the trackers 150 (e.g., the manipulator tracker 152 and the anatomy tracker 154) at specific times. By applying a derivative filter to the tracking data, the series of positions can be converted into velocities of the trackers 150. Even further, the derivative filter may also convert the velocities into acceleration values. At 412, a moving average filter may be applied to the tracking data. The moving average filter is generally used to reduce the amount of noise present in the tracking data. At 416, the system 100 may compensate for parameters of the camera/tracking device, such as by adjusting the tracking data based on one or more operational parameters of the localizer 110. This compensation step 416 may be implementation as described below in reference to a method shown in Figure 5. The computer 104 may have access to any number of operational parameters of the localizer 110, and the computer 104 may take the parameter(s) into account to convert the tracking data into the relative motions of the trackers 150.

At 420-428, like at 308-316 of the previous method 300, the relative motions of the trackers 150 are calculated based on the tracking data at step 420, the relative motions of the trackers 150 are compared to one another at 424, and the movement of the localizer 110 (e.g., the localizer motion parameter) is determined based on the comparison at 428.

At 432, the movement of the localizer 110 may be normalized, such as by normalizing the localizer motion parameter. In some implementations, the localizer motion parameter is normalized based on a velocity of the manipulator 204, such as the velocity of the manipulator 204 determined based on tracking data associated with the manipulator tracker 152. Normalizing the localizer motion parameter based on the manipulator velocity can increase the range of tracker velocities that can be handled by the method 400. In other words, the movement of the localizer is compared against a threshold movement following step 432, and normalizing the movement of the localizer based on the velocity of the manipulator 204 ensures that the method 400 responds the same way (e.g., leads to the same result at steps 436 and 440) when the manipulator 204 is moving at a higher velocity compared to when the manipulator 204 is moving at a lower velocity. More specifically, if the manipulator 204 is moving at a higher velocity and the localizer 110 is bumped slightly, the difference between the velocities of the manipulator 204 and the anatomy tends to be larger. On the other hand, if the manipulator 204 is moving at a lower velocity and the localizer 110 is bumped, the difference between the velocities may be smaller. Thus, to ensure the method 500 response consistently as mentioned above, the movement of the localizer 110, as determined at step 428, may be normalized based on the velocity/speed of the manipulator 204, such as by dividing the localizer motion parameter by the tracker motion parameter associated with the manipulator tracker 152.

At 436, the localizer motion parameter is compared against a threshold, such as a threshold localizer motion parameter. If the localizer motion parameter is below the threshold, the method 400 returns to 404. If the localizer motion parameter is above the threshold, however, the method continues to 440. At 440, a warning/notification is triggered to indicate that the localizer 110 has moved. The warning may include a visual shown on one of the displays 106, 107, an audible alert, a command sent to the manipulator 204 (e.g., a stop command), or any suitable alternative. The warning may also cause the system 100 to stop tracking the trackers 150, and/or instruct the user that the localizer 110 must be recalibrated to avoid tracking errors caused by the detected motion of the localizer 110 (i.e., caused by the movement of the localizer 110/the localizer motion parameter). Additionally or alternatively, the warning may cause the surgical robot 200 to limit further movement of the manipulator 204.

Referring to Figure 5, a flowchart describing a method 500 for detecting motion of the localizer 110 according to yet another implementation is shown. The method 500 includes the steps of the methods 300, 400 described above, and further (optionally) integrates kinematic data and data normalization to reduce the risk of false positive localizer motion warnings triggered by the system 100.

Between 504 and 528, the method 500 is largely the same as steps 404 to 428 of the method 400 shown in Figure 4. At 504, the method 500 begins with tracking data being received. For example, the localizer 110 may send the tracking data to the navigation computer 104 and the computer 104 may receive this data. At 508 and 512, derivative and/or moving average filters may be applied to the tracking data to aid in calculating the relative motion of each tracker 150 later in the method 500. At 516, the system 100 may compensate for parameters of the camera/tracking device in ways similar to step 416 of the method 400 shown in Figure 4. As described below, the method 500 may include analyzing kinematic data, such as data from the surgical robot 200, in combination with the tracking data. In such implementations, the tracking data may be captured by the localizer 110, and the localizer 110 may be configured to capture data at a first frequency, such as 60 Hz. At the same time, the kinematic data may be captured by the robot 200, and the robot 200 may be configured to capture data at a second frequency, such a 4 kHz, which is different from the first frequency. Further, the sources of the tracking/kinematic data, such as the localizer 110 and the robot 200, may store data to memory, such as memory available to the navigation computer 104, at the first and second frequencies, respectively. Even further, as the number of trackers 150 being tracked by the localizer 110 increases, the computing time needed to accurately determine the pose of the trackers may also increase and cause the tracking data to be captured at a third frequency, which is lower than the first and second frequencies.

In light of the difference in data capture/storage rate, the compensation step 516 may be carried out to ensure that the tracking data and kinematic data being used throughout the method 500 correspond to movement of the trackers 150 and movement of the manipulator 204 which occurred at the same time. One implementation of step 516 includes sending tracking data from the localizer 110 to the navigation computer 104, determining a first data travel time based on how long it took for the tracking data to be received by the navigation computer 104, sending kinematic data from the robot 200 to the navigation computer 104, determining a second data travel time based on how long it took for the kinematic data to be received by the navigation computer 104, and calculating a latency period by subtracting the second travel time from the first travel time. The latency period may then be considered when calculating tracker motion parameters at step 520.

At 520, after receiving the tracking data and optionally applying various filters and modifiers to the tracking data, the relative motion of each tracker 150, such as the tracker motion parameters associated with each of the trackers 150, is calculated based on the tracking data. In implementations that include the compensation step 516, the latency period may be considered when calculating the tracker motion parameters. For example, the navigation computer 104 may determine the localizer motion parameter corresponding to movement of the localizer at a first time, and determine a kinematic motion parameter (described below) corresponding to movement of the manipulator 204 at the first time. The localizer motion parameter may be based on tracking data captured at a second time, and the second time may be after the first time by a time period equal to the latency period. This ensures that the tracking data corresponds to movement of the tracker(s) 150 which occurred at the first time but was otherwise computed/received/stored in memory at the second time. After the relative motion of the trackers 150 are calculated, the method 500 may include comparing the relative motion of at least one of the trackers 150 to a minimum motion value at 522. The minimum motion value may correspond to a minimum relative motion of at least one of the trackers 150, as perceived by the localizer 110, that is detected when the localizer 110 is bumped/moved. If the relative motion of the tracker(s) 150 is below the threshold minimum motion value, the method 500 may conclude that the localizer 110 has not moved, that the movement of the localizer 110 was negligible and will not affect tracking performance, or that the manipulator 204 was not in use, and return to 504. This can help reduce the amount of false positives identified when carrying out the method 500. For example, the navigation computer 104 may know an average motion of the tracker(s) 150 caused by the user tapping the display 106, and the minimum motion value may be set based on this average motion. If the navigation computer 104 determines that the movement of the manipulator tracker 152 (and/or the anatomy tracker 154) is below the average motion caused by the user tapping the display 106, such as 5mm/s, the computer 104 can conclude that the movement of the manipulator tracker 152 was not caused by movement of the localizer 110. In another example, the minimum motion value corresponds to a minimum relative motion of the manipulator tracker 152 (and thus, the manipulator 204 itself). In this example, if the relative motion of the manipulator tracker 152 is below the threshold minimum motion value, the method 500 concludes that the manipulator 204 is not in use and that the localizer motion was either zero or low enough so as not to affect tracking performance. Regardless of implementation, if the relative motion of the tracker(s) 150 is above the threshold, the method 500 may continue to 524.

At 524, the relative motion of the trackers 150 are compared to one another. Subsequently, the movement of the localizer 110, such as the localizer motion parameter, is determined based on the relative motions of the trackers 150 at 528. After 528, however, the method 500 may include optional steps which introduce kinematic data, such as that generated by the surgical robot 200, to refine the output of the method 500. Stated differently, at least some of the steps found in the method 500 shown in Figure 5, but otherwise omitted from the methods 300, 400 shown in Figures 3 and 4, may be utilized by the system 100 in order to reduce the chance of a false positive warning being triggered at 568. To this end, at 532, kinematic data describing the motion of the manipulator 204 is received.

After the kinematic data has been received, such as by the navigation computer 104, the method 500 continues to 536. At 536 and 540, derivative and/or moving average filters may be applied to the kinematic data. In implementations in which steps 508 and 536 are applied to the tracking and kinematic data, the derivative filters applied at each step 508, 540 may have identical filter settings to ensure that the tracking data is filtered in the same way as the kinematic data. Similarly, in implementations in which steps 512 and 540 are applied to the tracking and kinematic data, the moving average filters may also have identical filter settings. If the filter settings are different, such as between the filters applied at steps 508 and 536 or the filters applied at steps 512 and 540, the tracking data may be filtered differently from the kinematic data. This difference in filtering can introduce inconsistencies between the two types of data which were not present prior to the filtering steps. Additionally or alternatively, the filter settings may affect the amount of time the computing system (e.g., the navigation computer 104) must spend applying the algorithm to the data. This may introduce a time lag between how fast the tracker motion parameters are computed versus how fast kinematic motion parameters (described below) are computed. In some methods involving data from multiple sources, a data fusion step may be applied to avoid this time lag. In the present method 500, instead of adding a data fusion step, the time lag may be eliminated or minimized by ensuring that the filter settings of each derivative filter are identical, as well as ensuring that the filter settings of each moving average filter are identical. After optionally filtering the kinematic data, at least one kinematic motion parameter is calculated at 544.

In implementations of the method 500 that include steps 532 through 544, step 528 may be carried out after step 544. Further, step 528 may be carried out using the kinematic motion parameter in addition to the manipulator motion parameter. In one case, the manipulator motion parameter may be compared to the kinematic motion parameter to determine a similarity metric. The similarity metric may then be compared against the anatomy motion parameter to determine the localizer motion parameter. More specifically, the similarity metric may be multiplied by the relative motion of the anatomy tracker 154 to determine the similarity of the two. For example, if the localizer 110 has moved while tracking the manipulator 204, the manipulator motion parameter will be different from the kinematic motion parameter. So, if the tracking data associated with the manipulator tracker 152 indicates that the manipulator 204 has moved 10mm/s upwards, and the kinematic data indicates that the manipulator 204 has moved 5mm/s upwards, the similarity metric will be determined as 5mm/s upwards via subtraction. The similarity metric of 5mm/s upwards may then be compared to the anatomy motion parameter as represented by the tracking data associated with the anatomy tracker 154. If the anatomy motion parameter indicates that the anatomy tracker 154 moved 5mm/s upwards as well, the localizer motion parameter may be determined as 5mm/s downwards. Additionally or alternatively, the similarity metric of 5mm/s upwards may be multiplied by the anatomy motion parameter of 5mm/s upwards, such as by finding a dot product of the two motion vectors (i.e., the two motion vectors of 5mm/s upwards). Since the magnitude of the dot product of two vectors is directly related to a similarity of the directions of each vector, the result of this multiplication will be higher where the relative motion of the anatomy tracker is similar to the difference between the relative motion of the manipulator tracker and the kinematic motion parameter. The higher the similarity, the higher the result of the multiplication operation, and the higher the chance that the localizer moved. At the same time, for the purposes of triggering warnings in response to the localizer motion parameter, the magnitude of the multiplication operation is directly related to the chance that the localizer motion parameter is determined to be above the threshold later on in the method 500. That said, if the anatomy motion parameter indicates that the anatomy tracker 154 has not moved, this may mean that the discrepancy between the manipulator motion parameter and the kinematic motion parameter was caused by something other than motion of the localizer 110, such as the manipulator tracker 152 moving relative to the manipulator 204.

As mentioned above, additional optional steps may be included in the method 500, and one or more of these steps may be performed to avoid false positives, which include triggering warnings when it would be unnecessary to do so. More specifically, at 548, the localizer motion parameter may be normalized based on the tracker motion parameter(s). For example, the localizer motion parameter may be divided by one of (1) the velocity of the manipulator based on kinematic data, (2) the velocity of the manipulator based on motion of the manipulator tracker 152, or (3) the average of these manipulator velocities. Like described above with respect to step 432 of the method 400, normalizing the localizer motion parameter based on the manipulator velocity can increase the range of tracker velocities that can be handled by the method 500.

In situations in which the patient is being tracked using the anatomy tracker 154, the method 500 may include determining whether the anatomy tracker 154 is visible (i.e., within a field of view of the localizer 110) at 552. If not, the method 500 may skip to 568 and a tracker visibility error may be triggered. The tracker visibility error may also be presented to the user, such as on the display 106. If the anatomy tracker is visible, however, the method 500 may continue to 556. At 556, the method 500 may include determining whether the relative motion of the anatomy tracker 154, such as the anatomy motion parameter, is above a threshold anatomy motion. In many cases, patient movement (i.e., anatomy tracker 154 movement) is not preferred, or is detrimental to the accuracy of the tracking data overall. Thus, if the relative motion of the anatomy tracker 154 is above the threshold, the method 500 may skip to 568 and a patient movement error may be triggered. The patient movement error may also be presented to the user, such as on the display 106. On the other hand, if the relative motion of the anatomy tracker 154 is below the threshold anatomy motion, the method 500 may continue to 560.

At 560, the localizer motion parameter may be scaled by an anatomy motion ratio, the anatomy motion ratio being equal to the relative motion of the anatomy tracker 154 divided by the threshold anatomy motion. For example, if the anatomy tracker 154 moved at 6mm/s and the threshold anatomy motion is 10mm/s, the localizer motion parameter is multiplied by 0.6. Scaling the localizer motion parameter by the anatomy motion ratio improves the reliability of the method 500. More specifically, the lower the relative motion of the anatomy tracker 154, the lower the anatomy motion ratio, and the lower the likelihood that localizer motion caused the relative motion of the anatomy tracker 154 (as perceived by the localizer 110). Further, the lower the anatomy motion ratio, the lower the localizer motion parameter after step 560. After scaling the localizer motion parameter by the anatomy motion ratio, the method 500 continues to 564.

After the movement of the localizer 110, such as the localizer motion parameter, has been normalized and/or scaled according to the steps described above, or if these steps were skipped, the method 500 may conclude with checking to see if the (optionally normalized and/or scaled) movement of the localizer 110 is above a predefined threshold at 564. If the movement of the localizer 110/ localizer motion parameter is below the threshold, the method 500 returns to 504 and restarts or ends. If the movement of the localizer 110/ localizer motion parameter is above the threshold, however, the method proceeds to 568. At 568, a warning/notification may be triggered in response to determining that the localizer motion parameter is above the threshold. The warnings/notifications may be like those described in reference to step 436 of the method 400 shown in Figure 4.

Referring to Figures 3-5 generally, it is further contemplated that certain steps of the methods 300, 400, 500 may be implemented in ways more specific than described above. A number of these implementations are described below.

Each of the methods 300, 400, 500 include calculating tracker motions parameters and a comparison between these tracker motion parameters. In some implementations, the comparison step 312, 424, 524 includes calculating a similarity metric, and the localizer motion parameter determination step 316, 428, 528 includes using the similarity metric to determine the localizer motion parameter. The similarity metric may be calculated by performing mathematical operations on at least one of the tracker motion parameters. For example, where the method 300, 400, 500 is being carried out using the manipulator and anatomy trackers 152, 154, the similarity metric may be calculated by subtracting the manipulator motion parameter from the anatomy motion parameter. In this example, the closer the similarity metric is to zero, the higher the similarity between the two motion parameters. Further, the higher the similarity between the two motion parameters, the higher the chance that the motion parameters were caused by movement of the localizer 110. Aside from subtraction, the similarity metric can be calculated based on a Euclidean distance calculation, a cosine similarity calculation, a dot product calculation, or any other suitable mathematical function.

As noted above, the similarity metric may also be utilized during the localizer motion parameter determination step 316, 428, 528. In other words, the localizer motion parameter may be determined based on the tracker motion parameters and the similarity metric. Expanding on the above example, the tracker motion parameters could be tracker velocities, the manipulator tracker 152 may have been moving at a velocity of 5mm/s in a first direction, and the anatomy tracker 154 may have also been moving at a velocity of 5mm/s in the first direction. Thus, the controller(s) carrying out the method 300, 400, 500 (e.g., the navigation computer 104) may calculate the similarity metric via subtraction to get a result of zero. In this case, the controller may determine the localizer motion parameter as a localizer velocity equaling 5mm/s in the first direction, because both trackers 152, 154 moved at a rate of 5mm/s in the first direction and the similarity metric indicated a high/exact similarity between the motion parameters of the trackers 152, 154. As another example, the controller may determine that the manipulator and anatomy trackers 152, 154 moved at 5mm/s in the first direction and 5mm/s in a second direction, respectively. In this example, the similarity metric may be calculated via subtraction to reach a result of 10mm/s in the first direction. Since the similarity metric is a relatively high number, especially compared to the tracker motion parameters, the controller may determine that the motion of the trackers 152, 154 was likely not caused by motion of the localizer 110.

The tracker motion calculation steps 308, 420, 520 may include the calculation of a plurality of motion parameters for each tracker 150 being tracked by the localizer 110, and the plurality of motion parameters (for each tracker 150) may be used during the remainder of the methods 300, 400, 500. In some implementations, the plurality of motion parameters may be a plurality of speeds, velocities, and/or accelerations of the trackers at various times. For example, the plurality of motion parameters could be a manipulator tracker velocity at a first time, a manipulator tracker velocity at a second time, an anatomy tracker velocity at the first time, and an anatomy tracker velocity at the second time. In this example, the motion parameter comparison steps 312, 424, 524 may includes multiple comparisons, such as a comparison of the manipulator tracker velocity at the first time and the anatomy tracker velocity at the first time, and another comparison of the manipulator tracker velocity at the second time and the anatomy tracker velocity at the second time. This way, the controller may determine whether the trackers 150 moved in a similar manner at multiple times, which may be a stronger indicator that the localizer 110 has moved compared to checking only a single motion parameter at a single time for the trackers 150. To streamline this process, the tracker motion calculation steps 308, 420, 520 may include calculating tracker velocity patterns for each of the trackers 150. The tracker velocity patterns may each include multiple tracker velocities at various times. To ensure that the motion parameters are comparable during the method 300, 400, 500, each tracker velocity pattern may include the same time frame during which the velocities were determined. In other words, if a first tracker velocity pattern includes velocities at a first time, a second time, and a third time, a second tracker velocity pattern may include velocities at the first, second, and third times as well. The comparison step 312, 424, 524 may then be carried out by comparing the tracker velocity patterns.

In the description above, reference is made to determining and/or calculating positions of the trackers 150 and the manipulator 204, such as in the methods 300, 400, 500. For the position of the manipulator 204, such as according to the tracking data associated with the manipulator tracker 152 and the kinematic data generated by the surgical robot 200, this may more specifically include the position of the TCP of the end effector 210 or surgical instrument 250. As such, any reference to the position of the manipulator 204 may include the position of the TCP. Further, any reference to relative motion, velocity, acceleration, speed, or motion parameter of the manipulator 204 may be understood as relative motion, velocity, acceleration, speed, or motion parameter of the TCP associated with the end effector 210 or surgical instrument 250.

Several implementations have been discussed in the foregoing description. However, the implementations discussed herein are not intended to be exhaustive or limit the invention to any particular form. The terminology which has been used is intended to be in the nature of words of description rather than of limitation. Many modifications and variations are possible in light of the above teachings and the invention may be practiced otherwise than as specifically described.

The many features and advantages of the invention are apparent from the detailed specification, and thus, it is intended by the appended claims to cover all such features and advantages of the invention which fall within the true spirit and scope of the invention. Further, since numerous modifications and variations will readily occur to those skilled in the art, it is not desired to limit the invention to the exact construction and operation illustrated and described, and accordingly, all suitable modifications and equivalents may be resorted to, falling within the scope of the invention.

## Claims

1. A surgical navigation system (100) comprising:
a first tracker (150, 152, 154);
a second tracker (150, 152, 154);
a localizer (110) configured to track the first tracker (150, 152, 154) to generate first tracking data and to track the second tracker (150, 152, 154) to generate second tracking data; and
one or more controllers (104, 208) being configured to:
receive the first tracking data and the second tracking data,
calculate a relative motion of the first tracker (150, 152, 154) based on the first tracking data,
calculate a relative motion of the second tracker (150, 152, 154) based on the second tracking data,
compare the relative motions of the first and second trackers (150, 152, 154) to one another, and
detect a movement of the localizer (110) based on the comparison.

2. The surgical navigation system (100) of claim 1, wherein the one or more controllers (104, 208) are configured to trigger a warning in response to detection of the movement of the localizer (110), and optionally, wherein the warning includes at least one of:
a visual on a display (106, 107),
an audible alert, and
a command sent to a robotic manipulator (200, 204).

3. The surgical navigation system (100) of claim 2, wherein the one or more controllers (104, 208) are configured to trigger the warning in response to the movement of the localizer (110) being above a threshold.

4. The surgical navigation system (100) of any one of claims 1 to 3, wherein:
the relative motion of the first tracker (150, 152, 154) is realized as a first tracker motion parameter;
the relative motion of the second tracker (150, 152, 154) is realized as a second tracker motion parameter;
the movement of the localizer (110) is realized as a localizer motion parameter; and
the one or more controllers (104, 208) are configured to:
compare the first tracker motion parameter to the second tracker motion parameter to calculate a similarity metric, and
determine the localizer motion parameter based on the first tracker motion parameter, the second tracker motion parameter, and the similarity metric.

5. The surgical navigation system (100) of claim 4, wherein:
the similarity metric is realized as a first similarity metric;
the first tracker motion parameter includes a first tracker velocity at a first time and a first tracker velocity at a second time;
the second tracker motion parameter includes a second tracker velocity at the first time and a second tracker velocity at the second time; and
the one or more controllers (104, 208) are configured to:
compare the first tracker velocity at the first time to the second tracker velocity at the first time to calculate the first similarity metric,
compare the first tracker velocity at the second time to the second tracker velocity at the second time to calculate a second similarity metric, and
determine the localizer motion parameter based on the first tracker motion parameter, the second tracker motion parameter, the first similarity metric, and the second similarity metric.

6. The surgical navigation system (100) of any one of claims 1 to 5, wherein:
the relative motion of the first tracker (150, 152, 154) includes a first tracker velocity pattern;
the relative motion of the second tracker (150, 152, 154) includes a second tracker velocity pattern; and
the one or more controllers (104, 208) are configured to compare the first tracker velocity pattern to the second tracker velocity pattern to detect the movement of the localizer (110).

7. The surgical navigation system (100) of any one of claims 1 to 6, wherein the one or more controllers (104, 208) are configured to:
calculate a first tracker velocity based on the first tracking data,
calculate a second tracker velocity based on the second tracking data,
compare the first tracker velocity to the second tracker velocity, and
detect the movement of the localizer (110) based on the comparison.

8. The surgical navigation system (100) of claim 7, wherein the one or more controllers (104, 208) are configured to:
calculate a similarity metric by subtracting the second tracker velocity from the first tracker velocity, and
detect the movement of the localizer (110) based on the first tracker velocity, the second tracker velocity, and the similarity metric.

9. The surgical navigation system (100) of any one of claims 1 to 8, wherein:
the first tracker (150, 152) is coupled to a robotic manipulator (200, 204);
the one or more controllers (104, 208) are configured to:
receive kinematic data from the robotic manipulator (200, 204),
calculate a kinematic motion parameter based on the kinematic data,
determine at least one localizer parameter corresponding to the localizer (110); and
calculate the relative motion of the first tracker (150, 152) based on the first tracking data and the at least one localizer parameter.

10. The surgical navigation system (100) of claim 9, wherein:
the at least one localizer parameter is a latency period;
the one or more controllers (104, 208) are configured to:
calculate the kinematic motion parameter based on kinematic data stored in memory at a first time, and
calculate the relative motion of the first tracker (150, 152) based on the first tracking data stored in memory at a second time; and the second time is after the first time by a time period equal to the latency period.

11. The surgical navigation system (100) of any one of claims 1 to 10, wherein:
the movement of the localizer (110) is realized as a localizer motion parameter;
the first tracker (150, 152) is coupled to a robotic manipulator (200, 204);
the relative motion of the first tracker (150, 152) is realized as a manipulator motion parameter; and
the one or more controllers (104, 208) are configured to:
receive kinematic data from the robotic manipulator (200, 204),
calculate a kinematic motion parameter based on the kinematic data, and
normalize the localizer motion parameter based on at least one of the kinematic motion parameter and the manipulator motion parameter.

12. The surgical navigation system (100) of any one of claims 1 to 11, wherein:
the movement of the localizer (110) is realized as a localizer motion parameter;
the localizer (110) includes a field of view;
the second tracker (150, 154) is realized as an anatomy tracker and is coupled to a bone of a patient;
the relative motion of the second tracker (150, 154) is realized as an anatomy motion parameter; and
the one or more controllers (104, 208) are configured to:
determine whether the anatomy tracker is within the field of view of the localizer (110) based on the second tracking data,
scale the localizer motion parameter based on the anatomy motion parameter in response to a determination that the anatomy tracker is within the field of view of the localizer (110), and
trigger a warning in response to a determination that the anatomy tracker is outside of the field of view of the localizer (110).

13. The surgical navigation system (100) of any one of claims 1 to 12, wherein the one or more controllers (104, 208) are configured to scale the movement of the localizer (110) based on the relative motion of the second tracker (150, 152, 154).

14. The surgical navigation system (100) of any one of claims 1 to 13, wherein:
the first tracker (150, 152) is coupled to a robotic manipulator (200, 204);
the relative motion of the first tracker (150, 152) is realized as a manipulator motion parameter; and
the one or more controllers (104, 208) are configured to:
receive kinematic data from the robotic manipulator (200, 204),
calculate a kinematic motion parameter based on the kinematic data,
calculate a similarity metric based on the manipulator motion parameter and the kinematic motion parameter,
compare the similarity metric against the relative motion of the second tracker (150, 152, 154), and
detect the movement of the localizer (110) based on the comparison.

15. A method (300, 400, 500) of operating a surgical navigation system (100) which includes a first tracker (150, 152, 154), a second tracker (150, 152, 154), a localizer (110) configured to track the first tracker (150, 152, 154) and the second tracker (150, 152, 154), and one or more controllers (104, 208), the method (300, 400, 500) comprising the one or more controllers (104, 208) performing the following:
receiving (304, 404, 504) a first tracking data associated with the first tracker (150, 152, 154) from the localizer (110);
receiving (304, 404, 504) a second tracking data associated with the second tracker (150, 152, 154) from the localizer (110);
calculating (308, 420, 520) a relative motion of the first tracker (150, 152, 154) based on the first tracking data;
calculating (308, 420, 520) a relative motion of the second tracker (150, 152, 154) based on the second tracking data;
comparing (312, 424, 524) the relative motions of the first and second trackers (150, 152, 154) to one another; and
detecting (316, 428, 528) a movement of the localizer (110) based on the comparison.
